# EUROPEAN PATENT SPECIFICATION

(11) **EP 4 558 273 B1**
(45) Date of publication and mention of the grant of the patent: **28.10.2026**
(21) Application number: 23843770.1
(22) Date of filing: 13.07.2023
(51) Int. Cl.: G01N 21/84, G01N 21/958, G01N 21/94, G01N 21/88, G06T 7/00, G16H 10/40, G16H 40/20, G16H 40/63

(54) **ANALYZER HAVING A TRANSPARENT SHIELD FOR PROTECTING AN IMAGING SYSTEM**
ANALYSATOR MIT EINER TRANSPARENTEN ABSCHIRMUNG ZUM SCHUTZ EINES BILDGEBUNGSSYSTEMS
ANALYSEUR DOTÉ D'UN ÉCRAN TRANSPARENT POUR PROTÉGER UN SYSTÈME D'IMAGERIE

(30) Priority: 18.07.2022 US 202263368681 P
(43) Date of publication of application: 28.05.2025
(73) Proprietor: Siemens Healthcare Diagnostics, Inc., Tarrytown, NY 10591 (US)
(72) Inventor: DESHPANDE, Manish, Newton, Massachusetts 02461 (US); ORTON, Emma, Cranston, Rhode Island 02905 (US)
(74) Representative: Dr. Müller Patentanwälte
(86) International application number: PCT/US2023/070092
(87) International publication number: WO 2024/020305

(56) References cited:
- WO-A1-2022/035846
- CN-A- 109 118 498
- DE-A1- 102013 101 888
- US-A1- 2011 136 109
- US-A1- 2011 223 673
- US-B1- 6 472 670

## Description

### CROSS REFERENCE TO RELATED APPLICATIONS/ INCORPORATION BY REFERENCE STATEMENT

### BACKGROUND

The inventive concepts disclosed herein generally relate to an analyzer having a transparent shield positioned between an optical reader and a sample holder, and more particularly, but not by way of limitation, to systems and methods configured to determine a degree of occlusion of the transparent shield while viewing a sample holder through the transparent shield.

To satisfy the needs of the medical profession as well as other expanding technologies, such as the brewing industry, chemical manufacturing, etc., a myriad of analytical procedures, compositions, and tools have been developed, including lateral flow immunoassays, and the so-called "dip-and-read" type reagent test devices. Regardless of whether lateral flow immunoassays, or dip-and-read test devices are used for the analysis of a biological fluid or tissue, or for the analysis of a commercial or industrial fluid or substance, the general procedure involves a test device coming in contact with the sample or specimen to be tested, and manually or instrumentally analyzing the test device.

A lateral flow immunoassay is a diagnostic device used to confirm the presence or absence of a target analyte. Lateral flow immunoassays typically contain a flow path which conveys a sample past a control line position and a test line position. A control line at the control line position confirms the test is working properly, and a test line at the test line position provides the result of the lateral flow immunoassay. Lateral flow immunoassays are developed to be used in a dipstick format or in a housed test format. Both dipsticks and housed tests work in a similar way, and generally fall within one of two categories: sandwich assays - a positive test is represented by the presence of a coloured line at the test line position; and competitive assays - a positive test is represented by the absence of a coloured line at the test line position.

Dip-and-read reagent test devices enjoy wide use in many analytical applications, especially in the chemical analysis of biological fluids, because of their relatively low cost, ease of usability, and speed in obtaining results. In medicine, for example, numerous physiological functions can be monitored merely by dipping a dip-and-read reagent test device into a sample of body fluid or tissue, such as urine or blood, and observing a detectable response, such as a change in color or a change in the amount of light reflected from, or absorbed by the test device.

Many of the dip-and-read reagent test devices for detecting body fluid components are capable of making quantitative, or at least semi-quantitative, measurements. Thus, by measuring the detectable response after a predetermined time, a user can obtain not only a positive indication of the presence of a particular constituent in a test sample, but also an estimate of how much of the constituent is present. Such dip-and-read reagent test devices provide physicians and laboratory technicians with a facile diagnostic tool, as well as with the ability to gauge the extent of disease or bodily malfunction.

Illustrative of dip-and-read reagent test devices currently in use are products available from Siemens Healthcare Diagnostics Inc., under the trademark MULTISTIX, and others. Immunochemical, diagnostic, or serological test devices, such as these usually include one or more carrier matrix, such as absorbent paper, having incorporated therein a particular reagent or reactant system which manifests a detectable response (e.g., a color change in the visible or ultraviolet spectrum) in the presence of a specific test sample component or constituent. Depending on the reactant system incorporated with a particular matrix, these test devices can detect the presence of glucose, ketone bodies, bilirubin, urobilinogen, occult blood, nitrite, and other substances. A specific change in the intensity of color observed within a specific time range after contacting the dip-and-read reagent test device with a sample is indicative of the presence of a particular constituent and/or its concentration in the sample. Some other examples of dip-and-read reagent test devices and their reagent systems may be found in U.S. Patents Nos. 3,123,443; 3,212,855; and 3,814,668.

However, dip-and-read reagent test devices suffer from some limitations. For example, dip-and-read reagent test devices typically require a technician to manually dip the test device into a sample, wait for a prescribed amount of time, and visually compare the color of the test device to a color chart provided with the test device. This process is slow and the resulting reading is highly skill-dependent (e.g., exact timing, appropriate comparison to the color chart, ambient lighting conditions, and technician vision) and may be inconsistent between two different technicians performing the same test. Finally, the act of manually dipping the test device into the sample may introduce cross-contamination or improper deposition of the test sample on the test device, such as via incomplete insertion of the test device into the sample, insufficient time for the sample to be deposited onto the test device, or having too much sample on the test device which may drip, leak, or splash on the technician's work area, person, or clothing.

Testing tools and methods have been sought in the art for economically and rapidly conducting multiple tests, especially via using automated processing. Automated analyzer systems have an advantage over manual testing with respect to cost per test, test handling volumes, and/or speed of obtaining test results or other information.

Automated instruments which are currently available for instrumentally reading individual reagent test devices, such as lateral flow immunoassays, or dip-and-read reagent test devices, or reagent strips, (e.g., CLINITEK STATUS reflectance photometer, manufactured and sold by Siemens Healthcare Diagnostics, Inc.) require each test device to be manually loaded into the automated instrument after contacting the test device with specimen or sample to be tested. Manual loading requires that the reagent test device be properly positioned in the automated instrument within a limited period of time after contacting the solution or substance to be tested. At the end of the analysis, used reagent test devices are removed from the instrument and disposed of in accordance with applicable laws and regulations.

Another development is the introduction of multiple-profile reagent cards and multiple-profile reagent card automated analyzers. Multiple-profile reagent cards are essentially card-shaped test devices which include multiple reagent-impregnated matrices or pads for simultaneously or sequentially performing multiple analyses of analytes, such as the one described in U.S. Pat. No. 4,526,753, for example. The reagent pads on the multiple-profile reagent card are typically arranged in a grid-like arrangement and spaced at a distance from one another so as to define several rows and columns of reagent pads. Adjacent reagent pads in the same row may be referred to as a test strip, and may include reagents for a preset combination of tests that is ran for each sample, for example.

Multiple-profile reagent cards result in an efficient, economical, rapid, and convenient way of performing automated analyses. An automated analyzer configured to use multiple-profile reagent cards typically takes a multiple-profile reagent card, such as from a storage drawer, or a cassette, and advances the multiple-profile reagent card through the analyzer over a travelling surface via a card moving mechanism, typically one step at a time so that one test strip (or one row of reagent pads) are positioned at a sample-dispensing position and/or at one or more read position. Exemplary card moving mechanisms include a conveyor belt, a ratchet mechanism, a sliding ramp, or a card-gripping or pulling mechanism. As the multiple-profile reagent card is moved or travels along the travelling surface and is positioned at the sample-dispensing position, one or more pipettes (e.g., manual or automatic) deposits a volume of one or more samples on one or more of the reagent pads on the reagent card. Next, the reagent pads are positioned at one or more read positions and analyzed (e.g., manually or automatically) to gauge the test result. The reagent card is placed in the field of view of an imaging system, such as an optical imaging system, a microscope, or a photo spectrometer, for example, and one or more images of the reagent pads on the card (e.g., optical signals indicative of the color of the reagent pads) is captured and analyzed. Typically, the field of view of the imaging system is relatively large to allow for the capture of multiple images of the same reagent pad as the reagent card is moved or stepped across multiple read positions in the field of view of the imaging system. The field of view encompasses multiple read positions or locations, and each reagent pad is moved in a stepwise fashion through the read positions as the reagent card travels across the field of view of the imaging system. Because the analyzer moves the card between various read positions in known intervals of time, the multiple images taken in the field of view of the imaging system allow the analyzer to determine changes in the color of the reagent pad as a result of the reagent pad reacting with the sample at each read position as a function of the time it takes the pad to be moved to the respective read position, for example. Finally, the used card is removed from the analyzer, and is disposed of appropriately.

Within some analyzers, a sample tray holds a consumable such as a reagent card to be read. The sample tray is moved by a motor from outside a housing of the analyzer to inside the housing where the sample measurement occurs by an optical reader. Surprisingly, excess fluid not captured by the consumable have been found to be splattered onto any optical components above the sample holding area during this movement. When optics become dirty the analyzer needs to be cleaned or replaced due to risk of an incorrect result that can be used by a doctor to treat a patient, which may require significant time and cost and potentially prevent results being available when needed. Examples of test analyzers with protective transparent shields are disclosed, for example, in DE102013101888 A1 and US2011223673 A1.

Accordingly, a need exists in the art for an analyzer having a sample tray that can be moved within the analyzer without contaminating optics of an optical reader within the analyzer. It is to such an improved analyzer that the present disclosure is directed to a transparent shield protecting the optical components of an imaging system, which may be removed, cleaned or replaced with convenience. Document CN109118498 A discloses the detection of stains or occlusions on a camera in dynamic-scene applications.

### SUMMARY

The present invention is directed to a method according to claim 1.

### BRIEF DESCRIPTION OF THE DRAWINGS

To assist those of ordinary skill in the relevant art in making and using the inventive concepts disclosed herein, reference is made to the appended drawings and schematics, which are not intended to be drawn to scale, and in which like reference numerals are intended to refer to the same or similar elements for consistency. For purposes of clarity, not every component may be labeled in every drawing. Certain features and certain views of the figures may be shown exaggerated and not to scale or in schematic in the interest of clarity and conciseness. In the drawings:
FIG. 1 is a front elevation view of an analyzer used in the inventive method, showing a transparent shield positioned in a field of view of an imaging system thereof.
FIG. 2 is a side elevation view of the analyzer of FIG. 1.
FIG. 3 is an end elevation view of the analyzer having the transparent shield positioned within a slot formed in a housing.
FIG. 4A is a bottom plan view of a circuit board having an aperture surrounded by onboard light sources to facilitate controlled illumination of the reagent card and reduce light scattering detected by the imaging system.
FIG. 4B is a side elevational view of a transparent shield positioned below a circuit board, the circuit board having an aperture surrounded by one or more illumination source.
FIG. 5 is top plan view image of a sample holder and test device as viewed through the transparent shield.
FIG. 6 is a top plan view image of the sample holder and test device as viewed through the transparent shield, wherein the transparent shield has an exemplary degree of occlusion based on material present on a first surface of the transparent shield
FIG. 7 is a top plan view image of the sample holder and test device as viewed through the transparent shield, wherein the transparent shield has a higher degree of occlusion than the top plan view image of FIG. 5.
FIG. 8 is a flow diagram of an exemplary embodiment of a method for determining a degree of occlusion of a transparent shield in accordance with the inventive concepts disclosed herein.
FIG. 9 is a bottom plan view of the transparent shield having an aperture.
FIG. 10 is a side elevation view of the transparent shield of FIG. 9 positioned ajar on a rail constructed and having an engaging member.
FIG. 11 is a side elevation view of the transparent shield engaging the rail and with the engaging member of the rail positioned within the aperture of the transparent shield of FIG. 9.
FIG. 12 is a top plan view of the transparent shield of FIG. 9 engaging the rail, the rail having the engaging member positioned within the aperture of the transparent shield.

### DETAILED DESCRIPTION

Before explaining at least one embodiment of the inventive concepts disclosed herein in detail, it is to be understood that the inventive concepts are not limited in their application to the details of construction and the arrangement of the components or steps or methodologies set forth in the following description or illustrated in the drawings.

In the following detailed description of embodiments of the inventive concepts, numerous specific details are set forth in order to provide a more thorough understanding of the inventive concepts. However, it will be apparent to one of ordinary skill in the art that the inventive concepts disclosed herein may be practiced without these specific details. In other instances, well-known features have not been described in detail to avoid unnecessarily complicating the instant disclosure.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherently present therein.

Unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present).

In addition, use of the "a" or "an" are employed to describe elements and components of the embodiments herein. This is done merely for convenience and to give a general sense of the inventive concepts. This description should be read to include one or at least one and the singular also includes the plural unless it is obvious that it is meant otherwise.

Further, as used herein any reference to "one embodiment" or "an embodiment" means that a particular element, feature, structure, or characteristic described in connection with the embodiment is included in at least one embodiment. The appearances of the phrase "in one embodiment" in various places in the specification are not necessarily all referring to the same embodiment.

As used herein "wet reagent test device" refers to a reagent device that has a volume of sample deposited thereon such that the reagent in the reagent device may react with its target constituent if such constituent is present in the sample. A wet reagent test device may also have a volume of a negative control deposited thereon.

As used herein, "reagent test device" refers to a carrier having a reagent. Exemplary reagent devices include a reagent pad of a dip and read test strip, or a control strip or a test strip of a lateral flow immunoassay.

Finally, as used herein qualifiers such as "about," "approximately," and "substantially" are intended to signify that the item being qualified is not limited to the exact value specified, but includes some slight variations or deviations therefrom, caused by measuring error, manufacturing tolerances, stress exerted on various parts, wear and tear, and combinations thereof, for example.

The inventive concepts disclosed herein are generally directed to a method according to claim 1. An analyzer for reagent test devices as described herein is used for carrying out the methods. The analyzer has a transparent shield in a field of view of an imaging system and a sample holder such that the imaging system is configured to capture images of the sample holder through the transparent shield. In some embodiments, the analyzer includes a processor. The processor is configured to receive an image and analyze pixels of the image to determine a degree of occlusion of the transparent shield. While the inventive concepts disclosed herein will be described primarily in connection with automatic analyzers using multiple-profile reagent cards as the reagent test device, the inventive concepts disclosed herein are not limited to automatic analyzers or to multiple-profile reagent cards. For example, a method according to the inventive concepts disclosed herein may be implemented with a manual analyzer, or may be implemented with an automatic analyzer using a reagent test device other than a multiple-profile reagent card, such as a lateral flow immunoassay, dip-and-read reagent test device, or a reel of reagent test devices on a substrate, and combinations thereof, as will be appreciated by a person of ordinary skill in the art having the benefit of the instant disclosure. Further, the inventive concepts disclosed herein may be implemented with any reagent device imaging system which has a field of view with at least one read position in the field of view.

In particular, a signal value indicative of a color of a reagent test device, such as a reagent pad, control line or test line, changes when the reagent test device is exposed to a sample. For a negative solution, the change in signal value is known (or can be measured) and therefore may become an optional offset signal value. Any change outside of the offset signal value is likely caused by a reaction with a clinical component that is being measured.

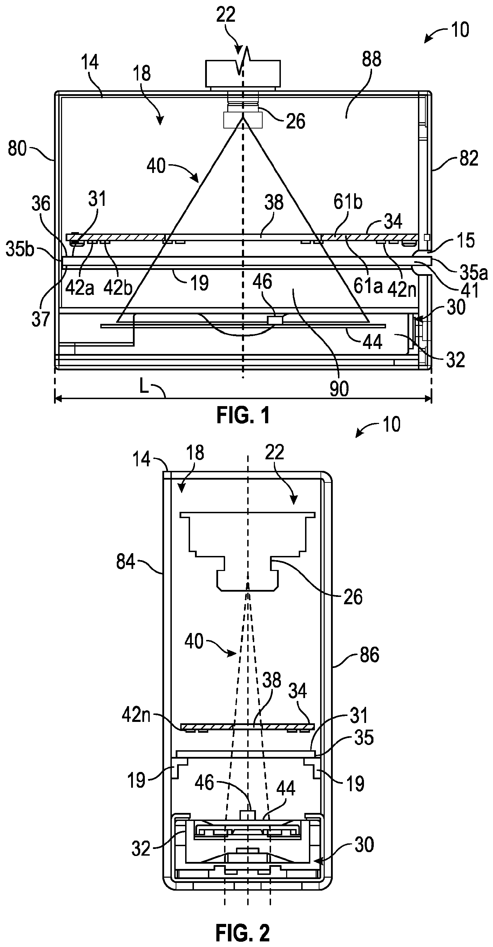

Referring now to FIGS. 1-3, shown therein is a reagent analyzer 10 according to the inventive concepts disclosed herein. The reagent analyzer 10 may be an automatic reagent card analyzer, for example. Exemplary embodiments of automatic reagent card analyzers are described in detail in US2013157372 A1, WO2013090655 A1.

Generally, the exemplary reagent analyzer 10 may include a housing 14, having a slot 15, the housing 14 surrounding a cavity 18. The reagent analyzer 10 also includes, at least one rail 19, an imaging system 22 comprising at least a camera 26, a sample tray 30 having a sample holder 32 positioned within the cavity 18, a transparent shield 31, and a circuit board 34 having an aperture 38 and one or more illumination source 42a-n positioned within the cavity 18.

The housing 14 may be formed from one or more components configured to form the cavity 18 and support the at least one rail 19, imaging system 22, the sample tray 30, the transparent shield 31, and the circuit board 34. In one embodiment, the housing 14 is opaque to visible light. In another embodiment, the housing 14 is opaque to one or more wavelength of light generated by the one or more illumination source 42a-n. In one embodiment, the housing 14 may normalize ambient light. In other non-limiting embodiments, the housing 14 has the slot 15 within which the transparent shield 31 may be positioned into the housing 14, and from which the transparent shield 31 may be removed from within the housing 14.

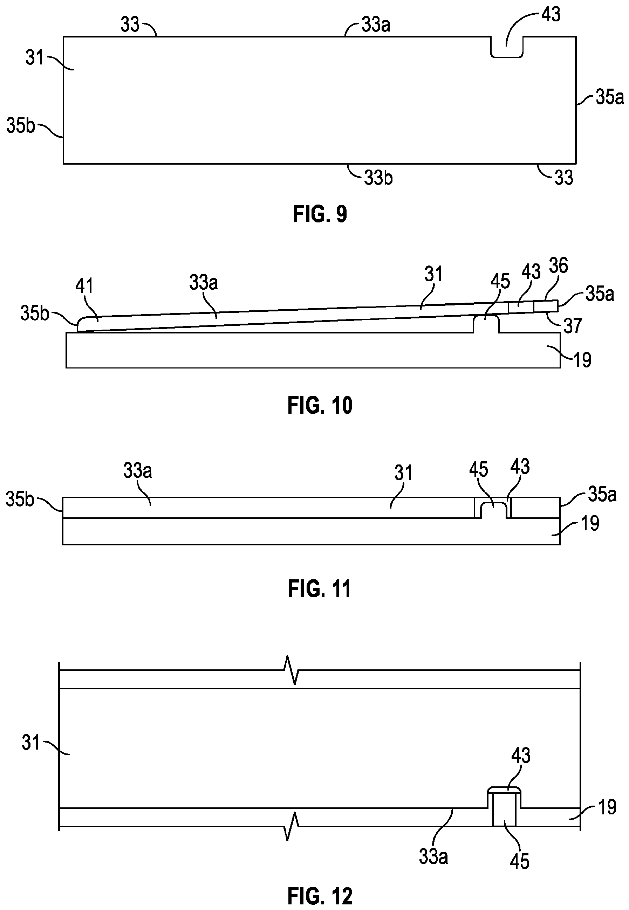

The transparent shield 31 has at least one sidewall 33, at least one end 35, a first surface 36 extending between the at least one sidewall 33 and the at least one end 35, a second surface 37 positioned opposite the first surface 36 extending between the at least one sidewall 33 and the at least one end 35, and an intermediate region 41 extending between the first surface 36 and the second surface 37. In one non-limiting embodiment, the transparent shield 31 has a first sidewall 33a, a second sidewall 33b positioned opposite the first sidewall 33a, a first end 35a, a second end 35b positioned opposite the first end 35a, the first surface 36 extending from the first end 35a to the second end 35b, a second surface 37 positioned opposite the first surface 36 extending from the first end 35a to the second end 35b, and the intermediate region 41 extending between the first surface 36 and the second surface 37. In one embodiment, the transparent shield 31 is transparent to visible light such that light may travel through the transparent shield 31 without appreciable scattering allowing objects positioned beyond the transparent shield 31 to be seen and imaged clearly. In some embodiments, the transparent shield 31 may have a degree of translucence such that light may travel through the transparent shield 31 with scattering allowing objects positioned beyond the transparent shield 31 to be seen with varying degrees of clarity. The first surface 36 and the second surface 37 may both be planar and substantially parallel so as to avoid magnifying visible light passing through the transparent shield 31. As will be discussed in more detail below, the transparent shield 31 is configured to protect the imaging system 22 from splatter or other debris resulting from movement of the sample tray 30 into and out of the housing 14. In some non-limiting embodiments, the transparent shield 31 may be movable within and out of the housing 14 through the slot 15. For example, the transparent shield 31 may have a grip (not shown) on at least one end 35 of the transparent shield 31. The grip may be a textured surface, e.g., a frost or an etching on the first surface 36 and/or the second surface 37, or may include a handle extending from and/or connected to the first surface 36 and/or the second surface 37, or the like. In some non-limiting embodiments, the transparent shield 31 has an aperture 43 positioned on an edge of the transparent shield 31 extending from the first surface 36 through the intermediate region 41 to the second surface 37. (FIG. 9).

In some non-limiting embodiments, the at least one rail 19 may be positioned within the cavity 18, adjacent to the slot 15 such that upon the positioning of the transparent shield 31 within the slot 15, the surface 37 of the transparent shield 31 may be positioned on the at least one rail 19. In other non-limiting embodiments, the at least one rail 19 may be positioned within the cavity 18, adjacent to the slot 15 such that upon positioning the transparent shield 31 within the slot 15, the surface 36 of the transparent shield 31 may be positioned on the at least one rail 19. In some non-limiting embodiments, the at least one rail 19 may have an engaging member 45 oriented in such a way that when the transparent shield 31 is being positioned within the cavity 18 of the engaging member 45 the causes transparent shield 31 to be in an ajar position. (FIG. 10). Upon the completion of the insertion of the transparent shield 31 into the cavity 18, the second surface 37 of the transparent shield 31 will be positioned on the at least one rail 19 and the engaging member 45 will be positioned within the aperture 43 of the transparent shield 31 (FIGS. 11 and 12). In order for a user to remove the transparent shield 31 from the cavity 18, an upward force may be applied to the second surface 37 of the transparent shield 31 so as to remove the engaging member 45 from the aperture 43 of the transparent shield 31. Upon the engaging member 45 being removed from within the aperture 43 the transparent shield 31 may be removed from the cavity 18. The transparent shield 31 may function as a barrier to prevent debris from the sample holder 32 from contacting the circuit board 34 and/or the imaging system 22.

The imaging system 22 includes the at least one camera 26 and is supported by the housing 14. In one embodiment, the imaging system 22 may be fixed to the housing 14 or fixed at a relative distance from the sample tray 30 or the transparent shield 31, for example. The imaging system 22 and/or the camera 26 may include one or more lens with a focal length selected to provide a field of view 40 to include at least the aperture 38 of the circuit board 34.

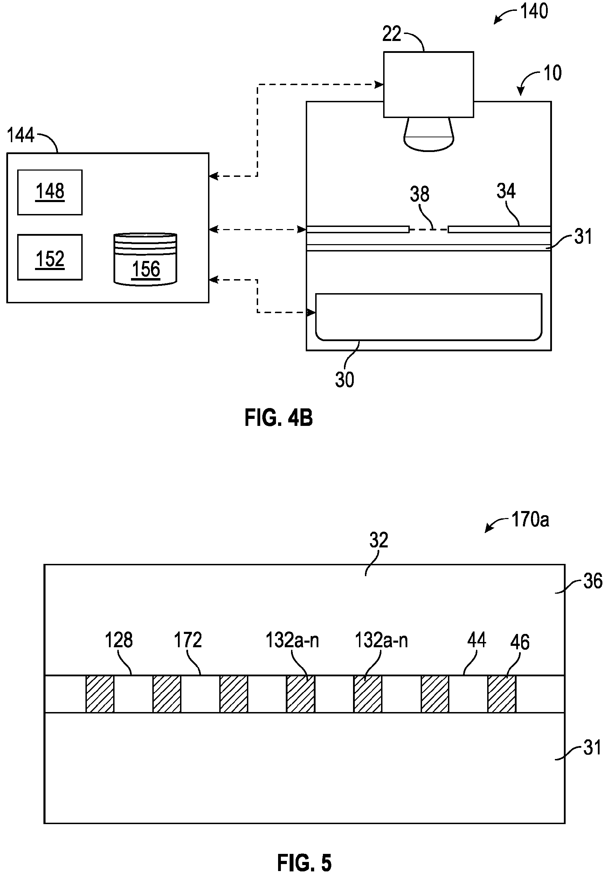

The imaging system 22 may be implemented and function as any desired reader such that the field of view 40 of the imaging system 22 includes substantially the entire aperture 38 of the circuit board 34, for example. The imaging system 22 may be supported at a location above, below, or beside the sample tray 30. In some embodiments, the field of view 40 may extend in a linear direction from the imaging system 22 to the aperture 38. In other embodiments, the field of view 40 may extend in a non-linear direction from the imaging system 22 to the aperture 38 due to the presence of one or more optical steering component in the field of view 40. Exemplary optical steering components include mirror(s), lens(es), beam splitter(s), or combinations thereof. The imaging system 22 may be configured to detect or capture an image or an optical signal indicative of a reflectance value or a color value of a reagent pad, a lateral flow assay, or the like, (shown in FIGS. 5-7 and discussed in more detail below) positioned in the field of view 40 of the imaging system 22, for example. In other non-limiting embodiments, the imaging system 22 may be configured to detect or capture an image or an optical signal indicative of a reflectance value or a color value of the sample holder 32 positioned in the field of view 40 of the imaging system 22, through the transparent shield 31. It is to be understood, however that in some exemplary embodiments, the field of view 40 of the imaging system 22 may include only a portion of the aperture 38 of the circuit board 34. It is also to be understood, that in some exemplary embodiments, field of view 40 of the imaging system may include only a portion of the transparent shield 31. The camera 26 of the imaging system 22 may include any desired digital or analog imager, such as a digital camera, an analog camera, a CMOS imager, a diode, and combinations thereof. The imaging system 22 may also include a lens system, optical filters, collimators, diffusers, or any other optical-signal processing devices, for example. Further, the imaging system 22 is not limited to an optical imager in the visible spectrum, and may include an infrared imaging system, an ultra-violet imaging system, a microwave imaging system, an X-ray imaging system, and/or other desired imaging systems, for example. Non-exclusive examples of the imaging system 22 include optical imaging systems, spectrophotometers, gas chromatographs, microscopes, infrared sensors, and combinations thereof, for example.

In one embodiment, the imaging system 22 includes at least one camera 26 and lens wherein the at least one camera 26 is an AR0239: CMOS Image Sensor, 2.3 MP, 1/2.7" and the lens is a DSL949 Sunex lens (Sunex Inc., Carlsbad, CA), both configured to maintain a large field of view 40 while keeping geometric image distortion low, thereby providing a resolution of 1080 pixels by 1920 pixels wherein each pixel depicts approximately a 0.065mm square area of the sample tray 30 and/or the sample holder 32.

The sample tray 30 may be configured to adjust the location of the sample holder 32 within the field of view 40. The sample holder 32 may be configured to receive at least one of test device 44, which may be a reagent card and a reagent card cassette, each having a sample 46. The sample 46 may be any bodily fluid, tissue, or any other chemical or biological sample, and combinations thereof, such as urine, saliva, or blood, for example. The sample 46 may be in liquid form and may contain one or more target constituents such as bilirubin, ketones, glucose, or any other desired target constituent, for example.

The circuit board 34 having the aperture 38 may be positioned within the cavity 18 and interposed between the imaging system 22 and the sample tray 30 such that the field of view 40 of the imaging system 22 is substantially unobstructed from the sample holder 32, the test device 44, and/or the sample 46. The circuit board 34 is described in FIGS. 1-2, and FIG. 4B and in more detail below. In one embodiment, the circuit board 34 is positioned at a fixed location between the imaging system 22 and the sample tray 30; however, in another embodiment, the circuit board 34 may be adjusted to varying locations between the imaging system 22 and the sample tray 30. If the circuit board 34 is adjustable, a calibration routine (described below) would have to be performed after any adjustment. In other non-limiting embodiments, the circuit board 34 may be positioned between the imaging system 22 and the transparent shield 31, such that the field of view 40 of the imaging system 22 is substantially unobstructed from the sample holder 32, the test device 44, and/or the sample 46.

Referring again to FIG. 1 and FIG. 2, the housing 14 may include a plurality of connected sidewalls 80, 82, 84 and 86 cooperating to surround the cavity 18. The sidewall 80 is spaced from the sidewall 82, and the sidewall 84 is spaced from the sidewall 86. The transparent shield 31 may be sized and dimensioned to traverse the cavity 18 between the sidewalls 80 and 82, and the sidewalls 84 and 86 thereby dividing the cavity 18 into a first portion 88 and a second portion 90. In some embodiments, the transparent shield 31 may have a length L between approximately 5 cm and approximately 21 cm. In some non-limiting embodiments, the transparent shield 31 may be a protecting device for the lens, wherein the transparent shield 31 may have a length of approximately 5 cm. In other non-limiting embodiments, the transparent shield 31 may be a protecting device for the lens and the optical components, wherein the transparent shield 31 may have a length of approximately 14 cm. In some non-limiting embodiments, wherein the transparent shield 31 is a protecting device for the lens and the optical components, the transparent shield 31 may not be removable from the reagent analyzer 10.

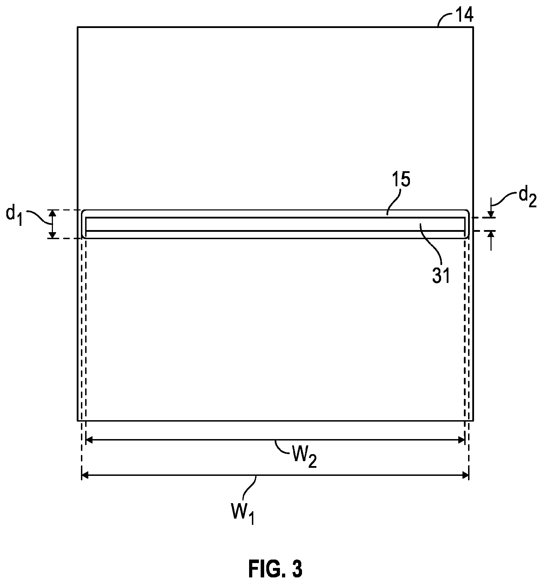

Referring to FIG. 3, shown therein is an end elevational view of the transparent shield 31 positioned within the slot 15 according to the inventive concepts disclosed herein. In some embodiments, the housing 14 has the slot 15, and the transparent shield 31 is positioned within the cavity 18 adjacent to the slot 15, the slot having a width W1 and a height d1, the transparent shield 31 having a width W2 less than the width W1 of the slot 15 and a thickness d2 less than the height d1 of the slot 15. In an exemplary, non-limiting embodiment the slot may have a width W1 of approximately 4.5 cm, a height d1 of approximately 0.2 cm, and the transparent shield 31 may have a width W2 of approximately 4.3 cm and a thickness d2 of approximately 0.1 cm. In some non-limiting embodiments, the height d1 of the slot 15 may be greater than 0.1 cm. In some non-limiting embodiments, the thickness d2 of the transparent shield 31 may be between approximately 0.1 cm and approximately .3 cm. In some embodiments, the width W1 of the slot 15 may be between approximately 1.7 cm and approximately 4.5 cm. In some non-limiting embodiments, the width W2 of the transparent shield 31 may be between approximately 1.5 and approximately 4.3 cm. In some embodiments, the transparent shield 31 is movably supported within the housing 14 and aligned with the slot 15 such that the transparent shield 31 is movable through the slot 15. In some embodiments, the surface 36 and the surface 37 of the transparent shield 31 are planar and parallel within the intermediate region 41 to avoid distorting or scattering light passing through the transparent shield 31. The transparent shield 31 may be separate from the imaging system 22 and configured to block debris originating from the sample tray 30 from coming into contact with the imaging system 22. The transparent shield 31 may be constructed of glass, ceramic, plastic, such as acrylic, polycarbonate, and the like.

The illumination source 42a-n may be implemented as one or more of a light emitting diode, a light bulb, a laser, an incandescent bulb or tube, a fluorescent light bulb or tube, a halogen light bulb or tube, or any other desired light source or object configured to emit an optical signal having any desired intensity, wavelength, frequency, or direction of propagation, for example. The illumination source 42a-n may be attached to the circuit board 34 and may be oriented such that substantially the entire field of view 40 of the imaging system 22 is illuminated by the illumination source 42a-n. In some exemplary embodiments, the illumination source 42a-n may be operably coupled with a controller 144 (see FIG. 4B - described in detail below) so that control and/or power signals may be supplied to the illumination source 42a-n by the controller 144. Desirably, the intensity of the optical signal emitted by the illumination source 42a-n is maintained substantially constant through the operation of the reagent analyzer 10, such as by control and power signals supplied by the controller 144. In one embodiment, the optical signals emitted by the illumination source 42a-n may be conditioned or processed by one or more optical or other systems (not shown), such as filters, diffusers, polarizers, lenses, lens systems, collimators, and combinations thereof, for example.

In some exemplary embodiments the one or more illumination source 42a-n may be implemented, such as a first illumination source 42a and a second illumination source 42b, and the first illumination source 42a and the second illumination source 42b may have different locations and/or orientations thereby causing the first illumination source 42a and the second illumination source 42b to cooperate to illuminate substantially the entire field of view 40 of the imaging system 22. (e.g., substantially the entire sample holder 32 and/or sample 46). The first illumination source 42a and the second illumination source 42b may emit optical signals having different illumination intensities, for example.

In one embodiment, the sample holder 32 may be adapted to accept the test device 44 in the form of a reagent card cassette having one or more multiple-profile reagent cards 124 therein, for example. An exemplary reagent card 124 is shown in FIGS. 5-7 and described in more detail below. Each reagent card 124 (detailed below) may include a substrate and one or more reagent pads positioned thereon, or otherwise associated therewith. In an exemplary embodiment, the reagent pads may include fluidic or microfluidic compartments (not shown).

Each reagent pad of the one or more reagent card positioned within the test device may include a reagent configured to undergo a color change in response to the presence of a target constituent such as a molecule, cell, or substance in the sample 46 of a specimen deposited on the reagent pad. The reagent pads may be provided with different reagents for detecting the presence of different target constituents. Different reagents may cause one or more color change in response to the presence of a certain constituent in the sample 46, such as a certain type of analyte. The color developed by a reaction of a particular constituent with a particular reagent may define a characteristic discrete spectrum for absorption and/or reflectance of light for that particular constituent. The extent of color change of the reagent and the sample 46 may depend on the amount of the target constituent present in the sample 46, for example.

The presence and concentrations of these target constituents in the sample 46 may be determinable by an analysis of the color changes undergone by the one or more reagent pads at predetermined times after application of the sample 46 to the reagent pads and/or at predetermined read positions in the field of view of the imaging system 22, for example. This analysis may involve a color comparison of each reagent pad to itself at different time periods after application of the sample 46 and/or at different read positions in the field of view 40 of the imaging system 22.

Based upon an analysis of a magnitude of the optical signal detected by the imaging system 22 the sample 46 may be assigned to one of a number of categories, e.g., a first category corresponding to no target constituent present in the sample 46, a second category corresponding to a small concentration of target constituent present in the sample 46, a third category corresponding to a medium concentration of target constituent present in the sample 46, and a fourth category corresponding to a large concentration of target constituent present in the sample 46, for example.

Further, the imaging system 22 may detect an optical signal indicative of a color or a reflectance value of a reagent pad and/or a test strip at any time interval after a volume of sample 46 has been dispensed on the test device 44, e.g., the reagent pad and/or test strip, and regardless of location of the particular reagent pad and/or test strip, for example. In one exemplary embodiment, a video, or a sequence of images may be captured of the reagent pad and/or test strip at a variety of time intervals after a volume of sample 46 is deposited on the reagent pad and/or test strip.

The imaging system 22 may be operated intermittently, continuously, or periodically, to detect one or more reflectance signals indicative of the color or the reflectance value of the one or more test devices 44, e.g., reagent pads, at any time and at any position in the field of view of the camera 26, for example. In some exemplary embodiments, the imaging system 22 may capture an image indicative of the color or the reflectance value of the test device 44, e.g., the reagent pad, prior to any sample 46 being deposited onto the reagent pad, or at any known time after a volume of sample 46 has been deposited onto the reagent pad, for example.

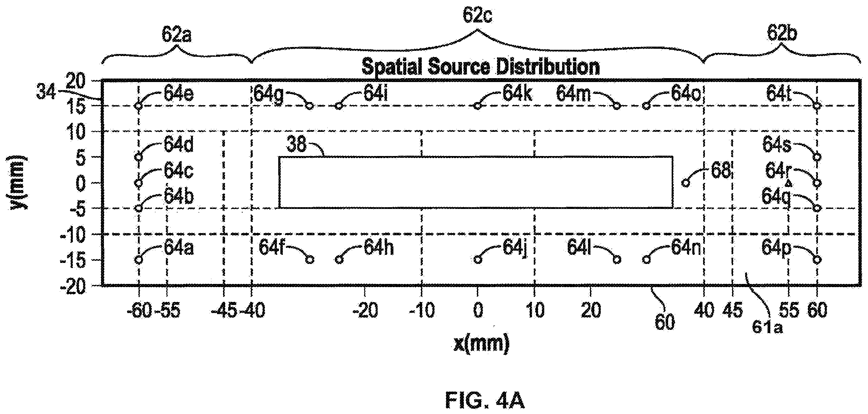

Referring now to FIG. 4A, shown therein is a bottom plan view of a circuit board having an aperture surrounded by onboard light sources according to the inventive concepts disclosed herein to facilitate controlled illumination of the reagent card and reduce light scattering detected by the imaging system 22.

Controlled illumination will be described herein by way of example as uniform illumination across an extent, i.e., length and width, of the sample holder 32 and/or sample 46 within acceptable limits. It should be understood, however, that the present disclosure is not limited to uniform illumination. The circuit board 34 is comprised of a substrate 60 having a bottom surface 61a and a top surface 61b, a plurality of conductive leads extending on or in the substrate 60, and the aperture 38 extending between the bottom surface 61a and the top surface 61b.

In one embodiment, shown in FIG. 4A, the one or more illumination source 42a-n is a plurality of LEDs 64a-n and one or more IR LED 68. The LEDs 64a-n shown in FIG. 4A include twenty (20) visible light LEDs arranged as shown in FIG. 4A, and one or more IR LED 68. The LEDs 64a-n includes any LED that is needed to produce a substantially uniform light intensity across the sample holder 32 and/or reagent card 124 or reagent cassette. The IR LED 68 may be used to apply heat to the sample 46, or identify an ID pad on the test device 44, for example. In one embodiment, the ID pad is utilized to correlate the sample 46 on the test device 44 supported by the sample holder 32 with a data obtained by the reagent analyzer 10.

In one embodiment, the plurality of LEDs 64a-n are selected to provide a fixed color, visible light, ultra-violet light, infrared light, or white light, or some combination thereof. In another embodiment, each LED 64a-n is positioned at an angle relative to the reagent card 124. In yet another embodiment, each LED 64a-n is positioned at one or more distance from the test device 44 supported by the sample holder 32 such that a first LED 64 and a second LED 64 are different distances from the test device 44 and/or the sample holder 32.

In some non-limiting embodiments, by adjusting the power level of each LED 64a-n, a substantially uniform light intensity may be achieved. The substantially uniform light intensity may be between 85% - 100% uniform.

The circuit board 34 as shown in FIG. 4A depicts the bottom surface 61a of the circuit board 34 having one or more illumination source 42a. As placed within the reagent analyzer 10, the bottom surface 61a is oriented to face the sample tray 30 such that light produced by the one or more illumination source 42a-n may be directly shown onto the sample holder 32 and/or sample 46. The illumination sources 42a-n are connected to the plurality of conductive leads of the circuit board 34 such that the conductive leads provide electricity to each illumination source 42a-n. In one embodiment, the circuit board 34 further includes illumination source circuitry (not shown) connected to the plurality of conductive leads and configured to apply electricity independently to each illumination source 42a-n. For example, the illumination source circuitry may be configured to supply a first power to a first illumination source 42a and a second power to a second illumination source 42b wherein the first power and the second power are different, thereby causing a difference in illumination intensity across the sample. The illumination sources 42a-n are arranged such that the illumination intensity across the field of view 40 of the camera 26 is substantially uniform, thereby increasing accuracy of readings of color changes of the reagent pads as the reagent pads are illuminated with a substantially uniform intensity (depicted in more detail below and in FIGS. 5-7). The substrate 60 of circuit board 34, as shown in FIG. 4B, is substantially planar thereby causing each of the one or more illumination source 42a-n to be a similar distance from the sample tray 30. Depending upon the location of the illumination source 42a-n relative to the sample 46, the distance between the illumination source 42a-n and the sample 46 may be different for certain of the illumination sources 42a-n. However, in other embodiments, the circuit board 34 may be non-planar thereby causing one of more of the illumination source 42a-n to be located at different distances from the sample tray 30. In one embodiment, one or more illumination source 42a-n may be affixed to a standoff (not shown) where each standoff is affixed to the circuit board 34 and provides one or more conductive paths to a particular one of the illumination source 42a-n. When a standoff is used, this causes a portion of the one or more illumination source 42a-n to be closer to the sample 46 and/or the sample tray 30.

In one embodiment, the substrate 60 has a first region 62a, a second region 62b opposite the first region 62a and an intermediate region 62c between the first region 62a and the second region 62b. The one or more illumination source 42a-n may be affixed to the substrate 60 in each of the first region 62a, second region 62b, and intermediate region 62c, or some combination thereof. In one embodiment, a first power may be applied to the one or more illumination source 42a-n within the first region 62a and within the second region 62b thereby causing the one or more illumination source 42a-n within the first region 62a and within the second region to provide a first illumination intensity and a second power may be applied to the one or more illumination source 42a-n within the intermediate region 62c thereby causing the one or more illumination source 42a-n within the intermediate region 62c to provide a second illumination intensity, the first power and the second power being different and the first illumination intensity and the second illumination intensity being different.

The aperture 38 of the circuit board 34 extends from the top surface 61b to the bottom surface 61a to provide an opening for the field of view 40 of the imaging system 22 to pass through from the imaging system 22 through the transparent shield 31 to the sample holder 32 and provide the camera 26 with a controlled view of the test device 44 associated with the sample holder 32. The aperture 38 may be further configured such that the bottom surface 61a of the circuit board 34 may include one or more illumination source 42a-n on each side of the aperture 38. In one embodiment, the aperture 38 is located substantially within the intermediate region 62c. In one embodiment, the aperture 38 has a first major axis and a first minor axis and the sample holder 32 has a second major axis and a second minor axis wherein the first major axis is aligned with the second major axis. While the aperture 38 is depicted as a rectangle in FIG. 4A for providing a controlled view of a rectangular reagent test device, it is understood that the aperture 38 may be configured of any shape such that the field of view 40 is a controlled view of the sample tray 30 and the illumination source 42 can be calibrated to provide a substantially uniform illumination of the sample 46. In the example of FIG. 4A, the aperture 38 does not extend to an edge of the circuit board 34.

In one embodiment, the aperture 38 extends to an edge of the circuit board 34 without bisecting the circuit board 34 whereas in another embodiment, the aperture 38 extends through the entire circuit board 34, bisecting the circuit board into a first half and a second half, wherein the first half and the second half are mounted at separate locations and supported by the housing 14 such that the field of view 40 is a controlled view of the sample tray and the illumination source 42.

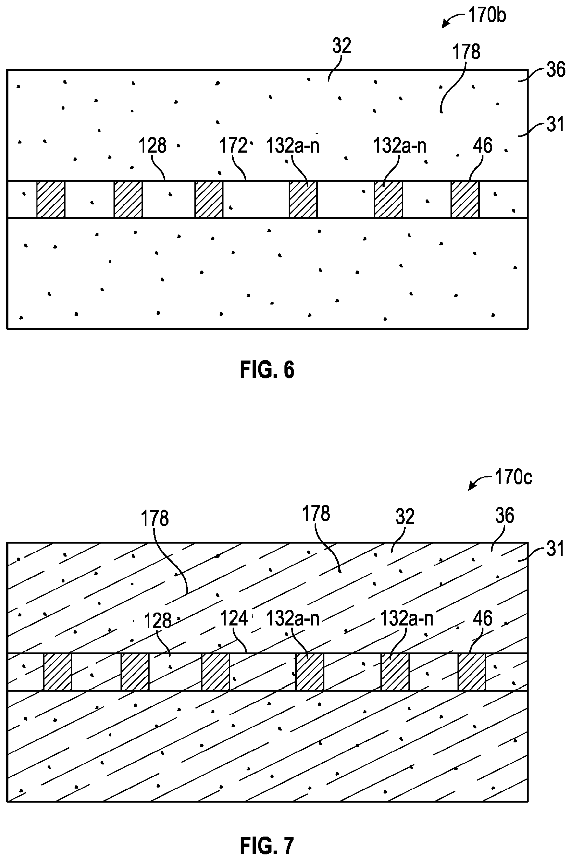

In some non-limiting embodiments shown in FIGS. 6-8, the reagent card 124 may include a substrate 128 and one or more, or a plurality of reagent pads 132a-n positioned thereon, or otherwise associated therewith. The substrate 128 may be constructed of any suitable material, such as paper, photographic paper, polymers, fibrous materials, and combinations thereof, for example. The reagent pads 132a-n may be arranged in a grid-like configuration on the substrate 128 so as to define one or more test strip, for example. In an exemplary embodiment, the reagent pads 132an may include fluidic or microfluidic compartments (not shown). The reagent pads 132a-n may be spaced apart a distance from one another so that the test strips are spaced apart such that adjacent test strips and/or reagent pads 132a-n may be simultaneously positioned at separate positions within the field of view 40 of the imaging system 22, for example. The reagent card 124 may be a multiple-profile reagent card having multiple reagent pads 132a-n having different reagents and/or multiple different test strips. Further, in some exemplary embodiments, the reagent card 124 may include one or more calibration chips or reference pads, which may have no reagent and may serve as color references, for example. In another embodiment, the reagent card 124 includes an ID pad having an identifier visible under IR light.

Each reagent pad 132a-n may include a reagent configured to undergo a color change in response to the presence of a target constituent such as a molecule, cell, or substance in the sample 46 of a specimen deposited on the reagent pad 132a-n. The reagent pads 132a-n may be provided with different reagents for detecting the presence of different target constituents. Different reagents may cause one or more color change in response to the presence of a certain constituent in the sample 46, such as a certain type of analyte. The color developed by a reaction of a particular constituent with a particular reagent may define a characteristic discrete spectrum for absorption and/or reflectance of light for that particular constituent. The extent of color change of the reagent and the sample may depend on the amount of the target constituent present in the sample 46, for example.

The color change may be read by the imaging system 22. Signals indicative of the color of the reagent pads 132a-n may be received and/or captured in an image by the imaging system 22, which may analyze the signals and determine a change in the color of the reagent pad 132a-n as a result of the reagent pad 132a-n reacting with the volume of sample 46 deposited thereon. Such color change may be analyzed as a function of the read position of the reagent pad 132a-n when the optical signal or image indicative of the color of the reagent pad 132a-n was detected and/or as a function of the known duration of time the volume of sample 46 has been deposited onto the reagent pad 132a-n, and combinations thereof, for example. The color change may be interpreted as a quantitative, qualitative, and/or semi-qualitative indication of the presence and/or concentration or amount of a target constituent in the volume of sample 46 deposited on the reagent pad 132a-n as described above.

Referring now to FIG. 4B, shown therein is an analyzer diagram 140 depicting the reagent analyzer 10 including the transparent shield 31 and an analyzer controller 144. The analyzer controller 144 has at least a processor 148 and a non-transitory computer readable memory 152. The memory 152 may store computer executable instructions that, when executed by the processor 148, causes the processor 148 to communicate with and/or be operable coupled to other elements of the reagent analyzer 10. While the analyzer controller 144 is depicted separately from the reagent analyzer 10, it is understood that in some embodiments, the analyzer controller 144 may be integrated into the reagent analyzer 10, such as, by way of example only, the analyzer controller 144 may be an additional component of the reagent analyzer 10 or may be integrated with another component of the reagent analyzer 10, for example, the circuit board 34.

In one embodiment, the imaging system 22 may be operably coupled with the analyzer controller 144 and/or the processor 148 so that one or more power and/or control signals may be transmitted to the camera 126 and/or to the one or more illumination source 42a-n by the controller 144, and so that one or more signals may be transmitted from the camera 126 to the processor 148, for example. The analyzer controller 144 may be configured to gauge test results as a reagent card is sampled within the reagent analyzer 10, for example, by receiving one or more signals from the camera 126. The camera 126 may be configured to detect or capture one or more optical or other signals through the transparent shield 31 that are indicative of a reflectance value of the test device 44, such as a reagent pad, and to transmit a signal indicative of the reflectance value of the test device 44, e.g., the reagent pad, to the processor, for example. One or more optical signals having wavelengths indicative of a reflectance value of the reagent pads and/or the test strip may be detected through the transparent shield 31 by the camera 126 at each read position, for example. The camera 126 may detect an optical signal through the transparent shield 31 indicative of a reflectance value of a reagent pad and/or test strip at any desired read position, location, or area within the field of view 40, or any other desired location or area or multiple locations or areas, for example. The signal transmitted to the processor 148 by the camera 126 may be an electrical signal, an optical signal, and combinations thereof, for example. In one embodiment, the signal is in the form of an image file having a matrix of pixels, with each pixel having a color code indicative of a reflectance value. In an exemplary embodiment, the image file may have two or more predetermined regions of pixels, each predetermined region of pixels corresponding to a read position of one of the reagent pads and/or the test strip in the field of view 40 of the camera 126. In one embodiment, the processor 148 may store the signal transmitted and or the image file in one or more database 156 and/or in the memory 152.

The processor 148 may determine the reflectance value or the color change of reagent pad and/or a test strip along with a sample (e.g., urine) disposed on the reagent pad and/or test strips based on the signals detected by the camera 126, for example. Each optical or other signal indicative of one or more reflectance value readings detected by the camera 126 may have a magnitude relating to a different wavelength of light (i.e., color). The color of the sample(s) and/or the reaction of the one or more reagents with a target constituent in a reagent pad may be determined based upon the relative magnitudes of the reflectance signals of various color components, for example, red, green, and blue reflectance component signals. For example, the color of each reagent pad may be translated into a standard color model, which typically includes three or four values or color components (e.g., RGB color model, including hue, saturation, and lightness (HLS) and hue, saturation, and value (HSV) representation of points and/or CMYK color model, or any other suitable color model) whose combination represents a particular color. In some embodiments the camera 126 may detect multiple optical signals at each read position, with each detected signal having one or more color components, such as a red component signal, a green component signal, and a blue component signal, for example, and each of the component signals may be transmitted to the processor 148. In some exemplary embodiments, the camera 126 may detect a single optical signal at each read position. and the processor 148 may translate a signal received from the camera 126 into separate color component signals such as a red component signal, a green component signal, and a blue component signal, for example.

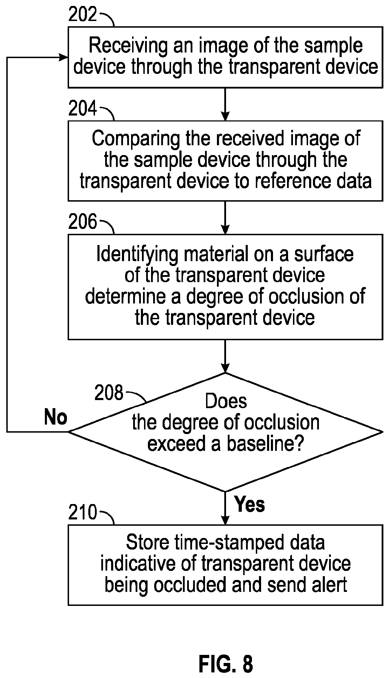

In one embodiment, the method for determining the degree of occlusion of the transparent shield 31 (described below and shown in FIG. 8) may be implemented as a set of processor executable instructions or logic stored in the non-transitory computer readable medium, which instructions or logic when executed by the processor 148, cause the processor 148 to determine the degree of occlusion of the transparent shield 31. The method for determining the degree of occlusion may be carried out periodically such as at a preset internal time as desired according to specific quality control procedures applicable to the reagent analyzer 10, and combinations thereof, for example.

In some embodiments, the processor 148 is further configured to have a set of processor executable instructions or logic stored in the non-transitory computer readable medium, wherein when the instructions or logic are executed by the processor 148, cause the processor 148 to cause an action on at least one periodic interval selected from a group consisting of: initiate an alert in a form perceivable by a human, initiate a cleaning process configured to clean the transparent shield 31, and replace the transparent shield 31. In some embodiments, the periodic interval is based on a period of time or a number of tests performed by the reagent analyzer 10.

Referring now to FIGS. 5-7, shown therein are exemplary images 170a-c illustrating a top plan view of the sample holder 32 and test device 44, in the form of a reagent card 172, as viewed through the exemplary transparent shield 31 positioned within the housing 14 of the reagent analyzer 10 in accordance with the present disclosure. In one embodiment, the imaging system 22 may capture the image 170 of the sample holder 32 and test device 44 through the exemplary transparent shield 31 having varying degrees of occlusion due to debris on the transparent shield 31 which may be caused by splatter from the test device 44 being moved by the sample holder 32.

FIG. 5 illustrates an exemplary top plan view image 170a of the sample holder 32 having the test device 44, in the form of a reagent card, as viewed through the transparent shield 31 that does not have an environmental agent 178 present on the surface 36 or surface 37 of the transparent shield 31.

FIG. 6 illustrates an exemplary top plan view image 170b of the sample holder 32 having the test device 44 in the form of the reagent card, as viewed through
the transparent shield 31 having environmental agents 178, such as dust, present on the surface 36 or the surface 37 of the transparent shield 31. Although many environmental agents 178 are shown in FIG. 6, only one environmental agent 178 is numbered for purposes of clarity.

FIG. 7 illustrates an exemplary top plan view image 170c of the sample holder 32 and test device 44, in the form of the reagent card 172, as viewed through an exemplary transparent shield 31 having many environmental agents 178, such as moisture, dust, and dirt, present on the surface 36 or the surface 37 of the transparent shield 31.

Referring now to FIG. 8, is a flow diagram of an exemplary embodiment of a method for determining the degree of occlusion of the transparent shield 31 in accordance with the inventive concepts disclosed herein. The method for determining the degree of occlusion of the transparent shield 31 generally includes the steps of: receiving a first image 170 of the test device 44 positioned within the sample holder 32 through the transparent shield 31 not associated with the imaging system 22, the transparent shield 31 positioned within the housing 14 of the reagent analyzer 10 and adjacent to the sample holder 32 (step 202); comparing pixel data of the received first image of the test device 44 positioned within the sample holder 32 through the transparent shield 31 to reference data stored in a non-transitory computer readable medium to determine a degree of occlusion of the transparent shield 31 (step 204); identifying material on a surface of the transparent shield 31 to determine the degree of occlusion of the transparent shield 31 (step 206); determining whether the degree of occlusion of the transparent shield 31 exceeds a baseline value (stored in the non-transitory computer readable memory 152) indicative of potential occlusion of the transparent shield 31 (step 208); and responsive to the degree of occlusion exceeding the baseline value, storing time-stamped data indicative of the transparent shield 31 being occluded, and causing an action selected from a group consisting of: initiating an alert in a form perceivable by a human, storing data in the non-transitory computer readable memory 152 indicative of the degree of occlusion detected within the first image 170 exceeding the baseline value, initiating a cleaning process configured to clean the transparent shield 31, replacing the transparent shield 31 with a replacement transparent shield 31 having a degree of occlusion less than the baseline value (step 210). In one non-limiting embodiment, the reference data may be data of the manufacturing standard of the test device 44 stored in a non-transitory computer readable medium.

In one embodiment, the transparent shield 31 may not be associated with the imaging system 22. In this embodiment, the primary function of the transparent shield 31 is to provide protection to the imaging system 22 and not to influence the optical characteristics of the imaging system 22. The transparent shield 31 may not be a lens or a component of the imaging system 22. In some embodiments, the transparent shield 31 may function to protect the imaging system 22, and also influence the optical characteristics of the imaging system 22. For example, the transparent shield 31 may include one or more polarizer or filter suitable to influence the optical characteristics of the imaging system 22. In either embodiment, the imaging system 22 may be calibrated, as discussed below, to reduce any inadvertent optical influence of the transparent shield 31.

Cleaning or replacing the transparent shield 31 improves the accuracy of the analysis provided by the reagent analyzer 10. In some embodiments, cleaning the transparent shield 31 can be implemented by moving the transparent shield 31 out of the housing 14 through the slot 15 (without the need to disassemble the analytical device or access the optical system directly), cleaning the surface 36 or the surface 37 of the transparent shield 31 and moving the transparent shield 31 into the housing 14 through the slot 15. Cleaning the transparent shield 31 can be implemented manually by a human gripping the transparent shield 31, or in an automated fashion. In the automated version, a motor-driven wiper can be used to wipe and clean the surface 36 or the surface 37. In some embodiments, replacing the transparent shield 31 can be implemented by moving the transparent shield 31 out of the housing 14 through the slot 15, discarding the transparent shield 31, and moving a replacement transparent shield 31 into the housing 14 through the slot 15.

The data indicative of the degree of occlusion may be stored in the memory 152, database 156, the non-transitory computer readable medium, or the like. In other non-limiting embodiments, the data indicative of the transparent shield 31 having a degree of occlusion may have a time stamp. In other non-limiting embodiments, when the degree of occlusion of the transparent shield 31 does not exceed the baseline value the method is further comprises the processor 148 receiving a second image 170 of the sample holder 32 through the transparent shield 31, comparing the received image of the sample holder 32 through the transparent shield 31 to reference data; determining the degree of occlusion of the transparent shield 31; and determining whether the degree of occlusion of the transparent shield 31 exceeds the baseline value ; i.e., repeating steps 202-210 until the degree of occlusion of the transparent shield 31 has met or exceeded the baseline value.

The method for determining the degree of occlusion of the transparent shield 31 may be implemented as a set of processor executable instructions or logic stored in the non-transitory computer readable medium, which instructions or logic when executed by the processor 148, cause the processor 148 to carry out the logic to calculate or determine the degree of occlusion of the transparent shield 31. The method for determining the degree of occlusion of the transparent shield 31 may be carried out periodically, such as at a preset interval of time or may be according to specific quality control procedures applicable to the reagent analyzer 10, and combinations thereof, for example.

In some non-limiting embodiments, the method for determining the degree of occlusion may be via computer vision for determining the at least one environmental agent 178 on the surface 36 or the surface 37 of the transparent shield 31. In some non-limiting embodiments, the method of computer vision for determining the at least one environmental agent 178 on the surface 36 or the surface 37 of the transparent shield 31 may be via object detection. Object detection is a technique that includes training a neural network to determine the presence of the at least one environmental agent 178 on the surface 36 or the surface 37 of the transparent shield 31 within the image 170 captured by the imaging system 22. The method by which the neural network may recognize the environmental agent 178 may include extracting several candidate regions which may be an object to be detected from the image 170 by utilizing a conventional region proposal method; inputting the extracted candidate regions into the neural network for recognition and categorization; and employing a bounding box regression technique, for example, to determine the environmental agent 178; surrounding each environmental agent 178 with a bounding box, determining whether the aggregate environmental agents 178 surrounded by the bounding box, i.e., degree of occlusion exceeds the baseline value indicative of the potential occlusion of the transparent shield 31. In some non-limiting embodiments, the alert generated in response to the degree of occlusion exceeding the baseline value may be a notification of the location of the environmental agent 178 on the surface 36 or the surface 37 of the transparent shield 31 on an output device. The output device may be a tablet, computer device, or the like.

In other non-limiting embodiments, computer vision for determining the at least one environmental agent 178 on the surface 36 or the surface 37 of the transparent shield 31 may utilize object tracking techniques. Object tracking includes a trained neural network running on the processor 148 to analyze changes in the images over time. This can be accomplished by receiving a first image of the sample holder 32 through the transparent shield 31 captured at a first instant of time, the processor 148 receiving a second image of the sample holder 32 through the transparent shield 31 captured at a second instant of time, wherein the second image is received by the processor 148 subsequent the receiving of the first image; detecting the environmental agent 178 in the first image to generate a bounding box wherein the environmental agent 178 may be positioned within the bounding box; performing an object recognition of the environmental agent 178 within the bounding box; repeating these steps for the second image, and determining whether the second image has the environmental agent 178 present in the first image based on the predetermined object model.

In some non-limiting embodiments, the computer vision for determining the at least one environmental agent 178 on the surface 36 or the surface 37 of the transparent shield 31 may utilize semantic segmentation techniques. Semantic segmentation includes constructing a first 3D semantic model, wherein the 3D semantic model comprises the reagent analyzer 10 having the transparent shield 31 not having at least one environmental agent 178 present on the surface 36 or the surface 37; constructing a second 3D semantic model of the reagent analyzer 10, wherein the transparent shield 31 has at least one environmental agent 178 present, such as dust, soot, ash, pollen, smoke, moisture, and the like. The 3D semantic model is updated over time by segmenting the current frame to form a segmented frame. The segmented frame depicting the reagent analyzer 10 having the transparent shield 31 in use may be compared to the 3D model using heuristic rules or a Bayesian rules to form a current frame of the reagent analyzer 10.

In some non-limiting embodiments, the computer vision for determining the at least one environmental agent 178 on the surface 36 or the surface 37 of the transparent shield 31 may utilize instance segmentation techniques. In the instance segmentation machine-learning model, the processor 148 may be trained to simultaneously process an image of the sample holder 32 through the transparent shield 31 positioned within the housing 14 of the reagent analyzer 10, and detect the at least one environmental agent 178, classifying the at least one environmental agent 178 via the positioning of a segmented mask over the image of the sample holder 32 through the transparent shield 31, positioned within the housing 14 of the reagent analyzer 10. The segmented mask may be a per-pixel mask that identifies the pixels of the at least one environmental agent 178 present on the at least one surface 36 or the surface 37 of the transparent shield 31.

In some non-limiting embodiments, the processor 148 may be configured to process an image of the sample holder 32 through the transparent shield 31 having the degree of translucence positioned within the housing 14 of the reagent analyzer 10, and detect the degree of translucence of the transparent shield 31. In some embodiments, the analyzer 10 may be configured to calibrate the degree of translucence of the transparent shield 31 to a known transparency standard of the transparent shield 31 stored in the memory 152 to normalize or correct for any inadvertent optical influence caused by light passing through the transparent shield 31. In some non-limiting embodiments, the calibration of the degree of translucence of the transparent shield 31 may be conducted at periodic intervals during use of the analyzer 10 or by operator command.

In some non-limiting embodiments, the processor 148 is further configured have a set of processor executable instructions or logic stored in the non-transitory computer readable medium, wherein when the instructions or logic are executed by the processor 148, cause the processor 148 to cause an action on at least one periodic interval selected from a group consisting of: initiate an alert in a form perceivable by a human, initiate a cleaning process configured to clean the transparent shield 31, and replace the transparent shield 31. In some embodiments, the periodic interval is based on a period of time or a number of tests performed by the reagent analyzer 10.

## Claims

1. A method, comprising:
receiving (202) a first image (170) of a test device (44) positioned within a sample holder (32) through a transparent shield (31) not associated with an imaging system (22), the transparent shield positioned within a housing (14) of a reagent analyzer and adjacent to the sample holder;
comparing (204) pixel data of the received first image of the test device positioned within the sample holder through the transparent shield to reference data stored in a non-transitory computer readable medium (152) to determine (206) a degree of occlusion of the transparent shield;
determining (208) whether the degree of occlusion of the transparent shield exceeds a baseline value indicative of potential occlusion of the transparent shield; and
responsive to the degree of occlusion exceeding the baseline value, causing (210) an action selected from a group consisting of: initiating an alert in a form perceivable by a human; storing data indicative of the degree of occlusion detected within the first image exceeding the baseline value; initiating a cleaning process configured to clean the transparent shield; and replacing the transparent shield with a replacement transparent shield having a degree of occlusion less than the baseline value,
analyzing the first image by a processor (148) executing processor executable code stored in a non-transitory computer readable medium to determine the degree of occlusion of the transparent shield, wherein analyzing the first image by the processor executing processor executable code is defined further as analyzing pixels within the image for a predetermined color indicative of an environmental agent (178) present on a surface of the transparent shield.

2. The method of claim 1, wherein when the degree of occlusion of the transparent shield exceeds the baseline value, the method further comprises:
storing data indicative of the transparent shield having the degree of occlusion; and
communicating a notification to a user indicative of the degree of occlusion of the transparent shield.

3. The method of claim 2, wherein the data has a time stamp.

4. The method of claim 1, wherein when the degree of occlusion of the transparent shield does not exceed the baseline value, the method further comprises:
receiving a second image of the test device positioned within the sample holder through the transparent shield;
determining the degree of occlusion of the transparent shield; and
determining whether the degree of occlusion of the transparent shield exceeds a baseline value.

5. The method of claim 1, wherein determining the degree of occlusion is performed utilizing machine vision techniques.

## Patentansprüche

1. Verfahren, umfassend:
Empfangen (202) eines ersten Bildes (170) einer Testvorrichtung (44), die innerhalb eines Probenhalters (32) positioniert ist, durch eine transparente Abschirmung (31), die nicht mit einem Bildgebungssystem (22) assoziiert ist, wobei die transparente Abschirmung innerhalb eines Gehäuses (14) eines Reagenzanalysators und angrenzend an den Probenhalter positioniert ist;
Vergleichen (204) von Pixeldaten des durch die transparente Abschirmung empfangenen ersten Bildes der Testvorrichtung, die innerhalb des Probenhalters positioniert ist, mit Referenzdaten, die in einem nichtflüchtigen computerlesbaren Medium (152) gespeichert sind, um einen Verdeckungsgrad der transparenten Abschirmung zu bestimmen (206);
Bestimmen (208), ob der Verdeckungsgrad der transparenten Abschirmung einen Basislinienwert überschreitet, der eine potenzielle Verdeckung der transparenten Abschirmung angibt; und als Reaktion darauf, dass der Verdeckungsgrad den Basislinienwert überschreitet, Veranlassen (210) einer Aktion, die aus einer Gruppe ausgewählt ist, die besteht aus: Initiieren eines Alarms in einer Form, die von einem Menschen wahrnehmbar ist; Speichern von Daten, die angeben, dass der Verdeckungsgrad, der innerhalb des ersten Bildes detektiert wird, den Basislinienwert überschreitet; Initiieren eines Reinigungsprozesses, der ausgelegt ist zum Reinigen der transparenten Abschirmung; und Ersetzen der transparenten Abschirmung durch eine transparente Ersatzabschirmung mit einem Verdeckungsgrad, der kleiner als der Basislinienwert ist, Analysieren des ersten Bildes durch einen Prozessor (148), der prozessorausführbaren Code ausführt, der in einem nichtflüchtigen computerlesbaren Medium gespeichert ist, um den Verdeckungsgrad der transparenten Abschirmung zu bestimmen, wobei das Analysieren des ersten Bildes durch den Prozessor, der prozessorausführbaren Code ausführt, ferner als Analysieren von Pixeln innerhalb des Bildes auf eine vorbestimmte Farbe definiert ist, die indikativ für einen Umweltagenten (178) ist, der auf einer Oberfläche der transparenten Abschirmung vorhanden ist.

2. Verfahren nach Anspruch 1, wobei, wenn der Verdeckungsgrad der transparenten Abschirmung den Basislinienwert überschreitet, das Verfahren ferner Folgendes umfasst:
Speichern von Daten, die indikativ dafür sind, dass die transparente Abschirmung den Verdeckungsgrad aufweist; und
Kommunizieren einer Benachrichtigung an einen Benutzer, die indikativ für den Verdeckungsgrad der transparenten Abschirmung ist.

3. Verfahren nach Anspruch 2, wobei die Daten einen Zeitstempel aufweisen.

4. Verfahren nach Anspruch 1, wobei, wenn der Verdeckungsgrad der transparenten Abschirmung den Basislinienwert nicht überschreitet, das Verfahren ferner Folgendes umfasst:
Empfangen eines zweiten Bildes der Testvorrichtung, die innerhalb des Probenhalters positioniert ist, durch die transparente Abschirmung;
Bestimmen des Verdeckungsgrads der transparenten Abschirmung; und
Bestimmen, ob der Verdeckungsgrad der transparenten Abschirmung einen Basislinienwert überschreitet.

5. Verfahren nach Anspruch 1, wobei das Bestimmen des Verdeckungsgrads unter Verwendung von Techniken des maschinellen Sehens durchgeführt wird.

## Revendications

1. Procédé, comprenant les faits suivants :
recevoir (202) une première image (170) d'un dispositif d'essai (44) positionné à l'intérieur d'un porte-échantillon (32) à travers un écran de protection transparent (31) non associé à un système d'imagerie (22), l'écran de protection transparent étant positionné à l'intérieur d'un logement (14) d'un analyseur à réactifs et adjacent au porte-échantillon ;
comparer (204) des données de pixel de la première image reçue du dispositif d'essai positionné à l'intérieur du porte-échantillon à travers l'écran de protection transparent à des données de référence stockées dans un support non transitoire lisible par ordinateur (152) pour déterminer (206) un degré d'occlusion de l'écran de protection transparent ;
déterminer (208) si le degré d'occlusion de l'écran de protection transparent dépasse une valeur de base indicative d'occlusion potentielle de l'écran de protection transparent ;
et
en réponse au cas où le degré d'occlusion dépasserait la valeur de base, causer (210) une action sélectionnée parmi un groupe constitué des faits suivants : déclencher une alerte sous une forme perceptible par un humain ; stocker des données indicatives du cas où le degré d'occlusion détecté à l'intérieur de la première image dépasserait la valeur de base ; déclencher un processus de nettoyage configuré pour nettoyer l'écran de protection transparent ; et remplacer l'écran de protection transparent avec un écran de protection transparent de remplacement ayant un degré d'occlusion inférieur à la valeur de base, analyser la première image par le biais d'un processeur (148) exécutant un code exécutable par processeur stocké dans un support non transitoire lisible par ordinateur pour déterminer le degré d'occlusion de l'écran de protection transparent, dans lequel le fait d'analyser la première image par le processeur exécutant un code exécutable par processeur est défini en outre comme étant le fait d'analyser des pixels à l'intérieur de l'image pour trouver une couleur prédéterminée indicative d'un agent environnemental (178) présent sur une surface de l'écran de protection transparent.

2. Procédé de la revendication 1, dans lequel, lorsque le degré d'occlusion de l'écran de protection transparent dépasse la valeur de base, le procédé comprend en outre les faits suivants :
stocker des données indicatives du cas où l'écran de protection transparent aurait le degré d'occlusion ; et
communiquer une notification à un utilisateur indicative du degré d'occlusion de l'écran de protection transparent.

3. Procédé de la revendication 2, dans lequel les données ont une estampille temporelle.

4. Procédé de la revendication 1, dans lequel, lorsque le degré d'occlusion de l'écran de protection transparent ne dépasse pas la valeur de base, le procédé comprend en outre les faits suivants :
recevoir une seconde image du dispositif d'essai positionné à l'intérieur du porte-échantillon à travers l'écran de protection transparent ;
déterminer le degré d'occlusion de l'écran de protection transparent ; et
déterminer si le degré d'occlusion de l'écran de protection transparent dépasse une valeur de base.

5. Procédé de la revendication 1, dans lequel le fait de déterminer le degré d'occlusion est réalisé en utilisant des techniques de vision par machine.
